# EUROPEAN PATENT SPECIFICATION

(11) **EP 0 505 374 B1**
(45) Date of publication and mention of the grant of the patent: **23.04.1997**
(21) Application number: 90916875.9
(22) Date of filing: 16.10.1990
(51) Int. Cl.: A61K 9/12, A61K 9/00

(54) **VITAMIN-MINERAL TREATMENT METHODS AND COMPOSITIONS**
VERFAHREN SOWIE ZUSAMMENSETZUNGEN ZUR BEHANDLUNG MIT VITAMINMINERAL
PROCEDES ET COMPOSITIONS DE TRAITEMENT A BASE DE VITAMINES-MINERAUX

(43) Date of publication of application: 30.09.1992
(60) Divisional application: 96202765.2
(73) Proprietor: MAYOR PHARMACEUTICALS LABORATORIES,INC., Phoenix, AZ 85034 (US)
(72) Inventor: DEIHL, Joseph, A., Phoenix, AZ 85034 (US)
(74) Representative: MacGregor, Gordon
(86) International application number: US9005999
(87) International publication number: WO9206726

(56) References cited:
- US-A- 4 814 161
- US-A- 4 826 683
- US-A- 4 851 211
- US-A- 4 889 709
- US-A- 4 956 385
- DATABASE WPIL Derwent Publications Ltd., London, GB; AN 88-157192

## Description

This invention relates to the manufacture of a pharmacological composition for treatment of the common cold.

US Patent 4,525,341 discloses methods for administering vitamins to humans by spraying a liquid vitamin composition into the mouth. The vitamin(s) were present in these compositions in a diet supplemental amount. The sprayable compositions contained a breath freshener and, optionally, a flavouring agent. The liquid carrier was a non-toxic alcohol, or an aqueous alcohol or water.

Urbach described the clinical use of Vitamin A buccal tablets in the treatment of leukoplakia and lichen planus in Bull. Ass. Milit. Derm 6: 17, 1957.

The local therapy of oral leukoplakia with Vitamin A troches was described by Mulay and Urbach in AMA Arch. Dem. and Syph. 78: 637-58, 1958.

Eby, Davies and Halcomb reported that zinc gluconate lozenges, dissolved in the mouth every two wakeful hours after an initial double dose, shortened the average duration of common colds by about seven days. Problems with palatability, taste distortion, mouth irritation, emetic properties and nausea were reported. Antimicrobial Agents and Chemotherapy, Vol. 25, No. 1, Jan. 1985, p. 20-24. (See also US-A-4 956 385).

The intra-nasal introduction of diverse drugs has been suggested and tested, including Vitamin B12, metoclopramide (cancer chemotherapeutant), insulin, LHRH hormone (contraceptive), lidocaine (headache medicine), alpha-interferon and enviroxime (rhinoviricides) and measle and flu vaccines. Some of these treatments induced side effects such as irritation and nasal bleeding.

JP-A-63096123 discloses the administration, including spraying, of a vitamin A zinc salt for anti-inflammatory purposes.

US-A-4826683 discloses the administration by nasal spray of a mixture including vitamin C and a zinc compound as a decongestant.

Thus, the prior art discloses that vitamins and other medicinal compositions can be administered by mucosal absorption from a composition sprayed into the oral cavity and that nasal administration of certain similar compositions may be feasible.

The present invention is concerned with treatment of the common cold by spraying a pharmacological composition into the oral cavity.

The invention provides use of vitamin C in the manufacture of a pharmacological composition including as active agent a non-toxic zinc salt, for improving the effectiveness of the active agent in the treatment of the common cold by spraying the composition into the oral cavity, the composition including a non-toxic solvent for the vitamin C and the active agent.

The composition may be administered as described in US-A-4525341, including forming an airspray of the composition, spraying the composition into the oral cavity and repeating the spraying step at spaced periods throughout the waking hours. This method can be improved by establishing a desired total dose for administration during a daily dosage period, selecting a predetermined concentration of the composition which will provide an aliquot dosage and repeating administration during the daily dosage period, to deliver the desired total dose.

The invention will now be described with such particularity as to enable any person skilled in the pertinent art to practice the invention without extensive experimentation.

In the administration of biologically active agents, including drugs, medicines and other compositions which are administered for pharmacological effect, it is normally required that a minimum concentration of the agent be established in the body, e.g., in the tissue or blood stream, and that such minimum concentration be maintained for a predetermined time period, as required to obtain the desired effect. Typically, these requirements are determined by empirical tests and the test results translated to a dosage and a pattern of repetition of such dosages as required to maintain the desired minimum body concentration of the agent. According to the prior art, such timed aliquot dosages have been administered orally, intravenously, intradermally or by inhalation to ultimately provide the desired total dosage. However, according to my present knowledge, the regimen of timed aliquot doses, substantially spaced throughout the wakeful period has not been practiced via mucosal absorption of the agent from solutions thereof sprayed into the oral cavity.

This involves desired total dosages of a composition being predetermined by appropriate clinical studies in which the compositions are sprayed into the oral cavity. Similarly, suitable aliquot dosages and administration intervals are predetermined based on observed clinical effects, after the spray administration of such compositions, e.g. by blood and/or tissue analysis and similar techniques. Having so predetermined the concentration of the composition and the spray dosage and interval, the method can be practiced by persons having only limited medical training and even by the patients themselves.

To improve the palatability of the spray composition I preferably include suitable flavoring agents in the spray composition, which may include any suitable flavoring agents which are soluble in the spray compositions. For example, flavoring agents such as sucrose, fruit juices, e.g. orange, apple, grape and pineapple juices and concentrates, are effectively employed.

The liquid carrier in the spray is chosen with reference to chemical compatibility with the active agents, including the ability to dissolve such agents, and non-toxicity as well as secondary factors such as taste, volatility, etc. The solvents can include, for example, water, aqueous alcohols or 200-proof spirits.

The spray can be formed by any suitable technique, including conventional air pump atomizers and conventional compressed gas atomizers. The specific type of dispenser is not highly critical. The selection of specific atomizer apparatus can be made by routine experimentation by persons skilled in the art, having regard for this disclosure.

The present invention permits a reduction in severity of common colds by intraoral application of non-toxic zinc solutions which exhibit rhinoviricidal activity along with Vitamin C, which improves the effectiveness of such treatment.

### Working Examples

The following examples will serve to illustrate the practice of my invention to those skilled in the art and to identify the presently preferred embodiment of my invention.

### EXAMPLE I

This example illustrates the preparation of a suitable sprayable zinc-Vitamin C composition. Table 1 identifies the final compositions by ingredient and weight percentage.

**Table 1**

| Ingredients | Parts by Weight |
|---|---|
| Pharmaceutical grade water (carrier) | 54.53 |
| Ascorbic Acid | 1.64 |
| Sodium Bicarbonate | 0.14 |
| Glycerine | 6.8 |
| Potassium Sorbate | 0.0765 |
| EDTA | 0.0082 |
| Zinc Gluconate | 1.09 |
| L-Lysine | 0.55 |
| Glycine | 0.55 |
| Fruit Juice (Flavoring) | 0.27 |
| Sucrose (Flavoring) | 32.79 |
| Magnasweet (GA-110) (Flavor Enhancer) | 0.27 |
| Emulsifier (Tween-80) | 0.55 |
| Trace Bioflavonoids (Rutin, Hesperidan, 27 others) | 0.27 |
| Orange Flavoring | 0.068 |
| Peppermint Oil | 0.492 |

The water at 85-92° F is measured into a sterile stainless steel vat. The glycerine and potassium sorbate is added to the water. The ascorbic acid and bicarbonate are separately mixed in a portion of the solution from the vat to stabilize the ascorbic acid and this solution is then added to the main batch in the vat. The following ingredients are then added singly with stirring to obtain complete solution thereof before the next ingredient is added: EDTA, zinc gluconate, L-lysine/glycerine, fruit juice/sucrose, flavor enhancer.

In a separate stainless steel container, the bioflavonoids are added to the emulsifier with constant mixing. Peppermint oil and then the orange flavoring are added to the container and mixed. The contents of the separate container are then slowly added to the vat to form a creamy light viscous solution.

### EXAMPLE II

The procedure of Example II is repeated except that ascorbic acid is omitted from the composition.

### EXAMPLE III

The procedure of Example I is repeated except that zinc gluconate is omitted from the composition.

### EXAMPLE IV

The procedure of Example I is repeated except that both ascorbic acid and zinc gluconate are omitted from the composition.

### EXAMPLE V

The compositions of Examples I to IV are administered by spraying every two hours during wakeful periods into the mouths of patients having the symptoms of common colds. The total daily doses administered are approximately 300 mg. zinc gluconate in the compositions of Examples I and II.

After seven days of these treatments the results are:

| Spray Compositions | % Patients Asymptomatic |
|---|---|
| Ex. I | 85 |
| Ex. II | 75 |
| Ex. III | 60 |
| Ex. IV | 45 |

## Claims

1. Use of vitamin C in the manufacture of a pharmacological composition including as active agent a non-toxic zinc salt, for improving the effectiveness of the active agent in the treatment of the common cold by spraying the composition into the oral cavity, the composition including a non-toxic solvent for the vitamin C and the active agent.

2. Use according to Claim 1, wherein the composition includes a flavouring agent.

3. Use according to Claim 1 or 2, wherein the composition includes sodium bicarbonate.

4. Use according to Claim 1, 2 or 3, wherein the composition includes glycerine.

5. Use according to Claim 1, 2, 3 or 4, wherein the composition includes potassium sorbate.

## Patentansprüche

1. Verwendung von Vitamin C bei der Herstellung einer Arzneimittelzubereitung mit einem nichttoxischen Zinksalz als aktivem Bestandteil zur Verbesserung der Wirksamkeit des aktiven Bestandteils bei der Behandlung üblicher Erkältung durch Einsprühen der Zubereitung in die Mundhöhle, wobei die Zubereitung ein nichttoxisches Lösungsmittel für das Vitamin C und den aktiven Bestandteil enthält.

2. Verwendung nach Anspruch 1, wobei die Zubereitung einen Geschmackstoff enthält.

3. Verwendung nach Anspruch 1 oder 2, wobei die Zubereitung Natriumbicarbonat enthält.

4. Verwendung nach Anspruch 1, 2 oder 3, wobei die Zubereitung Glycerin enthält.

5. Verwendung nach Anspruch 1, 2, 3 oder 4, wobei die Zubereitung Kaliumsorbat enthält.

## Revendications

1. Utilisation de la vitamine C dans la fabrication d'une composition pharmaceutique incluant comme agent actif un sel de zinc non toxique, pour améliorer l'efficacité de l'agent actif dans le traitement du rhume courant en pulvérisant la composition dans la cavité orale, la composition incluant un solvant non toxique pour la vitamine C et l'agent actif.

2. Utilisation selon la revendication 1, dans laquelle la composition inclut un agent de sapidité.

3. Utilisation selon la revendication 1 ou 2, dans laquelle la composition inclut du bicarbonate de sodium.

4. Utilisation selon la revendication 1,2 ou 3, dans laquelle la composition inclut de la glycérine.

5. Utilisation selon la revendication 1, 2, 3 ou 4, dans laquelle la composition inclut du sorbate de potassium.
